Europäisches Patentamt

European Patent Office

Office européen des brevets

(11) Publication number: **0 482 303 A1**

# EUROPEAN PATENT APPLICATION

(21) Application number: **91113299.1**

(22) Date of filing: **08.08.91**

(51) Int. Cl.5: **A61M 3/02**

(30) Priority: **22.10.90 IT 2198390 U**

(43) Date of publication of application:
**29.04.92 Bulletin 92/18**

(84) Designated Contracting States:
**AT CH DE DK ES FR GB GR IT LI NL SE**

(71) Applicant: **Conte, Raffaele**
**Via Washington, 57/A**
**I-20146 Milan(IT)**
Applicant: **Civetti, Arturo**
**Via Giotto, 7**
**I-20145 Milan(IT)**

(72) Inventor: **Conte, Raffaele**
**Via Washington, 57/A**
**I-20146 Milan(IT)**
Inventor: **Civetti, Arturo**
**Via Giotto, 7**
**I-20145 Milan(IT)**

(74) Representative: **Dr. Ing. A. Racheli & C.**
**Viale San Michele del Carso, 4**
**I-20144 Milano(IT)**

(54) **Rapid enema apparatus, possibly medicated, with adjustable flow and interchangeable delivery taper collar.**

(57) An enema apparatus, possibly medicated, comprising a delivery taper collar (18), connected by means of a flexible hose (17) to a tank (4), in which an electric motor (10) is located, operated by a suction pump (11), to whose delivery duct (13) a flexible hose (17) is connected, a chamber (5) being located at the side of the tank (4), into which the delivery (13) of the pump (11) comes out, and intended to house the delivery taper collar (18) and the flexible hose (17) when the apparatus is not in use, as well as an adjustable transformer (23) for low voltage feeding of the motor (10).

FIG. 1

The present invention refers to an apparatus for intestinal washing, carried out for stimulating defecation.

Apparatuses of this type are commonly used, generally consisting of a tank into which the washing liquid is introduced, connected by means of a duct to a delivery taper collar, suitable for being inserted into the rectum. The tank is either in the same body which houses the delivery taper collar, or forms a separate body, connected to the delivery taper collar by means of a flexible hose. For the former type, a disadvantage is represented by the limited capacity of the tank, while in the latter type the tank may have a large volume, but it has to be hung by means of hooks or other fixing devices at a higher level with respect to the patient's position, in order to obtain a pressure sufficient for delivery. In addition, in order to facilitate the ejection of the washing liquid, the patient is almost forced to assume a prone position, usually lying on a bed. In addition, in both types of apparatus, it is not possible to adjust the delivery pressure, with the result that the operation is very long because of the particularly slow flow rate of the liquid.

It has also been proposed to connect the feed hose of the delivery taper collar directly to the water mains. In this case it is not possible, however, to carry out enemas of the so-called "medicated" type, that is with the addition to the water of substances stimulating defecation, such as camomile, mallow, petroleum oil and so on, and it is also necessary to keep a check on the water mains pressure.

The aim of the invention is to avoid the drawbacks of the systems described above, but keep the advantages of each of them, by providing an enema apparatus with a tank which has an adequate capacity, for example two litres, and is provided with an easily interchangeable delivery taper collar, which will deliver water, possibly medicated, at adjustable pressure, and which can be used autonomously and simply by the patient, even in the same place and the usual position for the normal act of defecation.

The apparatus according to the invention comprises a tank for the delivery of the liquid, in which an electric motor is located operating a suction pump, with the delivery taper collar feed hose connected to its delivery side.

The electric motor is fed from the mains by an adjustable voltage transformer, by means of which a first adjustment of the flow can be made. A second adjustment can be made by means of a tap positioned on the delivery duct of the pump, or on the delivery collar feed hose.

On one side of the tank, a chamber with an opening door is suitably made, in which the transformer is housed and the delivery taper collar is located, together with the flexible hose, when the apparatus is not in use.

Further characteristics of the enema apparatus according to the invention will be clearer to understand from the detailed description below, referring to one of its merely exemplary, and therefore not restrictive embodiments, shown in the appended drawings, in which:

Figure 1    is an axonometric diagrammatic view of the equipment according to the invention;

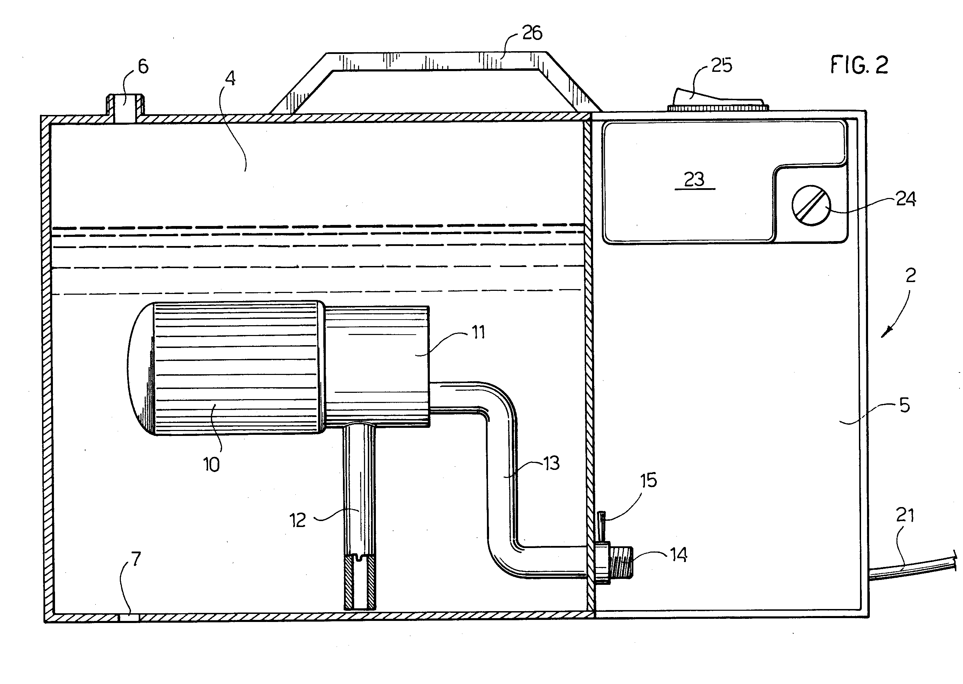

Figure 2    is a section taken along line II-II in figure 1.

With reference to such figures, the enema apparatus according to the invention is shown as a whole with reference number 1.

The apparatus comprises a parallelepiped box-shaped casing 2, divided by a watertight inner bulkhead 3 in two compartments 4 and 5, the first acting as a water tank and provided, for this purpose, with an upper filling hole 6 and a lower drain hole 7, both closed with respective plugs 8, 9.

An electric motor 10 is located in the tank 4 for operating a suction pump 11, having a suction duct 12 and an outlet or delivery duct 13, which crosses the separation bulkhead 3 and comes out in the chamber 5 with a threaded connector 14, in correspondence with which a tap 15 is located. To the connector 14 a corresponding complementary connector 16 is screwed, foreseen at the end of a flexible hose 17, made of rubber, latex or nylon, and at the other end a delivery taper collar 18 is applied by pressure or in any other way, made of non-toxic plastic, rubber or latex, hollow and interchangeable, with a slightly conical shape. Near the base of the delivery taper collar 18 a protective cover 19 with a concave profile is foreseen.

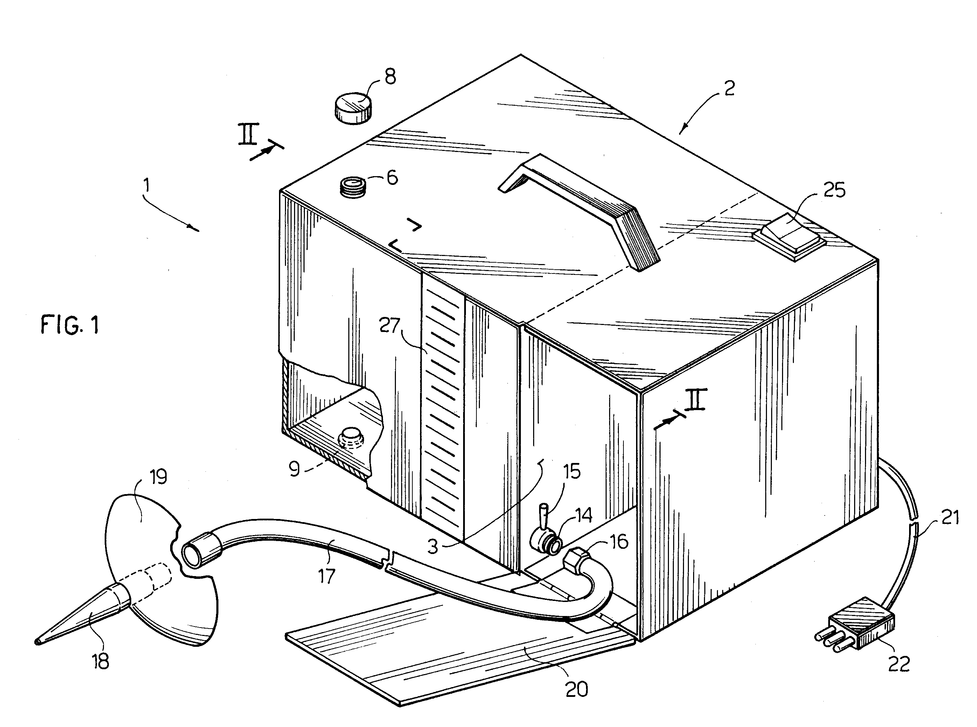

The delivery taper collar 18 and the flexible hose 17 are usually housed in the chamber 5, from which they are taken out at the moment they are used, by opening a door 20 at the front, which in figure 1 has, merely as an example, been foreseen hinged to the box 2, but which obviously can be opened in other ways, for instance by sliding, or even by being taken off.

An electric wire 21 is foreseen for feeding the electric motor 10, with a related plug 22 and an adjustable transformer 23, housed in the chamber 5.

Adjustment of the output voltage of the transformer 23 can be carried out by acting on a screw 24. In this way the first adjustment of the pump 11 delivery is obtained. A second adjustment is carried out, obviously, by means of the tap 15. On the upper wall of the casing 2 an ON/OFF switch 25 is foreseen together with a handle 26.

On the front wall of the tank 4 at least one vertical transparent strip is foreseen as a graduated scale 27 for checking the water level in the tank. Obviously, so-called "medicated" substances for stimulating defecation, such as camomile, sage, petroleum oil and the like, can be added to the water in the tank 4.

The working of the apparatus is made clear by the description given below.

From what has been described above, the advantages of the apparatus according to the invention are clear to see, in that it is easy to be handled and used by the patient, it has an adequate capacity, for example at least two litres, with the possibility of checking the level of the liquid, it allows flow, and therefore pressure, to be adjusted within certain limits, by acting on double adjustment means, consisting of the adjustment screw 24 of the transformer 23 and of the tap 15, and allows the delivery taper collar 18, to be changed simply and rapidly, and therefore be "personalized".

Of course, the invention is not limited to the particular embodiment previously described and illustrated in the appended drawings, but changes can be made to its details which are within the reach of a technician in the art.

In this way, for example, a compressor which blows along the free surface of the liquid in the tank 4 can be foreseen instead of the pump 11, submerged in the liquid, thus causing the latter to come out through the connector 14, with the advantage of eliminating almost all contact with the liquid.

The apparatus may also be foreseen with battery feed, for example of 12 volts, which is useful in the case of temporary absence of mains current, and/or to make the apparatus portable.

Finally, the apparatus according to the invention can be used, without substantial modifications, for vaginal douching, selecting a suitably medicated liquid.

**Claims**

1. An enema apparatus comprising a delivery taper collar (18) connected to a liquid tank (4) by means of a flexible hose (17), characterized in that in said tank (4) an electric motor (10) is foreseen, operating a suction pump (11), to whose delivery duct said flexible hose (17) is connected.

2. An apparatus according to claim 1, characterized in that at the side of said tank (4) there is a chamber (5), into which the delivery duct (13) of the pump (11) comes out, provided with a connector (14) for connecting the flexible hose (17), the chamber (5) being suitable for housing both the delivery taper collar (18) and the flexible hose (17) when the apparatus is not in use, and having an opening or removable door (20).

3. An apparatus according to claim 2, characterized in that a tap (15) is foreseen on said connector (14).

4. An apparatus according to any one of the previous claims, characterized in that said motor (10) is fed from the mains by means of an adjustable transformer (23), whose outlet voltage can be adjusted by acting on a screw (24), for adjusting the flow of the pump (11).

5. An apparatus according to any one of the claims from 1 to 3, characterized in that said motor (10) is fed by a battery.

6. An apparatus according to claim 4, characterized in that said transformer (23) is housed in the chamber (5).

7. An apparatus according to any one of the previous claims, characterized in that at least one vertical strip of one side wall of the tank (4) is transparent and has a graduated scale (27) for checking the liquid in the tank.

8. An apparatus according to any one of the previous claims, in which said tank (4) has an upper hole (6) for filling and a lower hole (7) for draining, closed respectively by plugs (8, 9).

9. An apparatus according to any one of the previous claims, characterized in that near the base of said delivery taper collar (18) a protective cap (19) is foreseen.

10. An apparatus according to any one of the previous claims, in which said pump (11) is replaced by a compressor which acts on the free surface of the liquid contained in the tank (4).

FIG. 1

EP 0 482 303 A1

FIG. 2

EP 0 482 303 A1

**European Patent Office**

# EUROPEAN SEARCH REPORT

Application Number

**EP 91 11 3299**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 800 840 (ABELL)<br>* page 7, line 16 - page 8, line 10 *<br>* page 9, line 23 - line 29 *<br>* page 10, line 11 - line 26 *<br>* page 12, line 16 - line 20; claims 1,5,16-18; figures 2,6,11 *<br>* | 1,5 | A 61 M 3/02 |
| X | EP-A-0 363 519 (LIU ET AL)<br>* column 1, line 37 - line 51 *<br>* column 3, line 5 - line 41 *<br>* abstract; figure 2 * * | 1 | |
| Y | | 2-9 | |
| Y | US-A-3 281 864 (LINNEHAN)<br>* column 2, line 20 - line 54 *<br>* column 3, line 33 - line 44; figures 1,1A,2 * * | 2-9 | |
| X | US-A-1 956 006 (COONS)<br>* the whole document * * | 10 | |
| A | | 3,8 | |
| A | US-A-1 853 202 (DE FOREST)<br>* page 1, line 75 - line 84; figures 1,2 * * | 9 | TECHNICAL FIELDS SEARCHED (Int. Cl.5)<br><br>A 61 M<br>A 61 H<br>A 61 C |

The present search report has been drawn up for all claims

| Place of search | Date of completion of search | Examiner |
|---|---|---|
| The Hague | 17 January 92 | GIMENEZ BURGOS R. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

 

& : member of the same patent family, corresponding document